(19) Europäisches Patentamt
European Patent Office
Office européen des brevets

(11) Publication number: 0 096 125 B1

(12) **EUROPEAN PATENT SPECIFICATION**

(45) Date of publication of patent specification: **07.01.87**

(51) Int. Cl.$^4$: **C 07 D 241/20,** C 07 D 241/16, A 61 K 31/495

(21) Application number: **82302976.4**

(22) Date of filing: **09.06.82**

# (54) Amino and amido derivatives of chloro nitro amino and alkylamino pyrazines useful as adjuncts to radiation therapy.

(43) Date of publication of application: **21.12.83 Bulletin 83/51**

(45) Publication of the grant of the patent: **07.01.87 Bulletin 87/02**

(84) Designated Contracting States: **AT BE CH DE FR GB IT LI LU NL SE**

(58) References cited:
**GB-A-1 230 908**
**GB-A-1 232 758**
**US-A-3 660 397**

(73) Proprietor: **MERCK & CO. INC.**
**126, East Lincoln Avenue P.O. Box 2000**
**Rahway New Jersey 07065 (US)**

(72) Inventor: **Hartman, George D.**
**1529 Tennis Circle**
**Lansdale Pennsylvania, 19446 (US)**

(74) Representative: **Crampton, Keith John Allen et al**
**D YOUNG & CO 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to substituted derivatives of 6-amino-3-chloro-5-nitropyrazinamino compounds and their acid-addition salts. Such compounds can be prepared by first converting 3-amino-5,6-dichloropyrazine carboxylic acid by nitrate in sulfuric/nitric acid to 5,6-dichloro-3-nitropyrazineamine and subsequently treating the latter in the presence of a base such as a tertiary amine to produce the desired 2-substituted compounds and, if desired, converting the 2-substituted pyrazines to their acid-addition salts. The present invention results from the discovery that certain such compounds can selectively sensitize tumor cells to therapeutic radiation and thus increase the effective therapeutic ratio of radiologic treatment.

Certain of our prior patent specifications disclose various 6-amino-3-chloro-5-nitropyrazinamino compounds that can be represented by the formula:

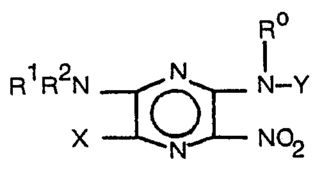

in which variables include those set out below in the various documents. Thus, in US—A—3 660 397, X is halogen, e.g. chlorine, each of $R^1$ and $R^2$ is hydrogen or $C_{1-6}$ alkyl, e.g. methyl or ethyl, $R^o$ is hydrogen, $C_{1-6}$ alkyl or ($C_{1-3}$ alkoxy)-substituted $C_{1-6}$ alkyl, e.g. methyl, 2-methoxyethyl or ethyl, and Y is hydrogen or lower alkanoyl; and in UK Patents GB—A—1 230 908 and GB—A—1 232 758, Y and $R^o$ are both hydrogen, X is chlorine, $R^2$- is $X^2$-$CHX^1$-, each of $R^1$ and $X^1$ is hydrogen or $C_{1-5}$ alkyl, e.g. ethyl, and $X^2$ is hydrogen, $C_{1-5}$ alkyl, ($C_{1-5}$ alkoxy)-substituted $C_{1-5}$ alkyl, di($C_{1-5}$ alkyl)amino-substituted $C_{1-5}$ alkyl or $C_{1-5}$ alkyl having an N-attached heterocyclic substituent, e.g. methyl, ethyl, *n*-propyl, iso-propyl, methoxymethyl, dimethylaminomethyl, diethylaminomethyl or dimethylamino-*n*-propyl. However, these prior patent specifications disclose that the compounds are useful as intermediate compounds in the production of therapeutically valuable imidazopyrazines, but not that the pyrazines themselves have any therapeutic utility.

Compounds that are suitable for use in enhancing the therapeutic effect of radiation treatment are those of formulae:

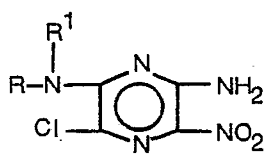

I

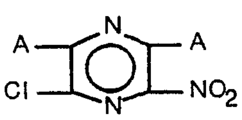

IA

and

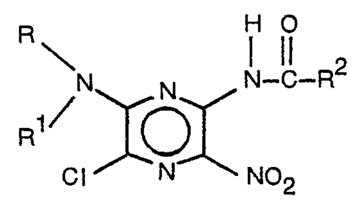

IB

and their acid-addition salts. In formula I, each of R and $R^1$ is hydrogen, $C_{1-6}$ alkyl, $C_{2-6}$ alkenyl, $C_{1-6}$ substituted alkyl or $C_{2-6}$ substituted alkenyl having one or more halo, amino, $C_{1-6}$ alkylamino, di($C_{1-6}$ alkyl)amino, $C_{1-6}$ alkoxy, hydroxy, ethoxycarbonyl, phenyl, pyridyl, pyrimidinyl or imidazoyl substituents and in Formula IB each of R and $R^1$ is hydrogen, $C_{1-6}$ alkyl, $C_{2-6}$ alkenyl, or a $C_{1-6}$ substituted alkyl or $C_{2-6}$ substituted alkenyl radical having one or more amino, $C_{1=6}$ alkylamino, di($C_{1-6}$ alkyl)amino, $C_{1-6}$ alkoxy or hydroxy substituents, or in the Formula I and IB, R and $R^1$ together form a morpholine, piperazine or N-substituted piperazine ring in which the N-substituent is hydrogen, $C_{1-6}$ alkyl, $C_{1-6}$ hydroxyalkyl or $C_{1-6}$ alkoxyalkyl; $R^2$ is $C_{1-6}$ alkyl; and A is an amino substituent having at least one of the hydrogens of the amino groups replaced by a hydroxy-substituted or ($C_{1-6}$ alkoxy)-substituted $C_{1-6}$ alkyl residue.

At the present time, certain other unrelated compounds are in experimental clinical use as radiation sensitizers. However, these compounds, for example, metronidazole and misonidazole, suffer from the drawback that they also cause symptoms of neurotoxicity which limits their usefulness. The compounds for use in the present invention are effective radiation sensitizers, but are believed to have a more favourable therapeutic ratio. The compounds for use in the present invention are preferably included in a pharmaceutical composition that also includes a pharmaceutical carrier.

Compounds or formula IA can be prepared by reaction of the corresponding 2-halo-5,6-dichloro-3-nitropyrazine with a suitably substituted alkyamine of formula AH to replace the 2-halo and 6-chloro substituents by the residue A. Compounds of formula IB can be prepared by reaction of 5,6-dichloro-3-nitropyrazinamine with an acylating agent derived from a suitable aliphatic carboxylic acid to afford the corresponding acylated pyrazinamine and treating the latter with a compound of formula $NHRR^1$ to replace

one of the chloro substituents and produce a 2-acylamino-6-($NRR^1$)-5-chloro-3-nitropyrazine. The acid-addition salts may be formed by reaction of the free amino compound with an equimolar amount of a strong mineral acid such as hydrochloric acid, hydrobromic acid, sulfuric acid or phosphoric acid.

The amines described above are prepared from the known 3-amino-5,6,-dichloropyrazine carboxylic acid (E. J. Cragoe, Jr., O. W. Woltersdorf, Jr., J. B. Bicking, S. F. Kwong, J. H. Jones, *J. Med. Chem., 10,* 66 (1967)). The carboxylic acid is dissolved in sulfuric acid or fuming sulfuric acid. To the solution formed in this manner, a mixture of equal volumes of sulfuric and nitric acid or the corresponding fuming sulfuric and fuming nitric acids are added to the solution to effect replacement of the carboxylic acid substituent by a nitro-substituent. The nitric and sulfuric acids are used in approximately equimolar amounts to the carboxylic acid reagent. Preferably, however, these acids are used in from 10—100% molar excess. The reaction temperature is maintained between 0 and 50°C for a period of 0.5 to 5.0 hours. The progress of the reaction is followed by observation of the evolution of carbon dioxide from the reaction mixture. This reaction is illustrated in the following reaction scheme:

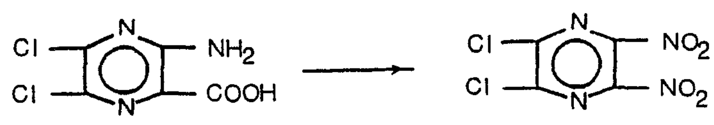

V II

The product is conveniently recovered from the reaction mixture by pouring the entire mixture onto ice whereupon the product is precipitated as a crude yellow solid. Further purification is effected by dissolving the crude product in a solvent such as ethyl acetate and washing with an alkaline solution such as aqueous sodium carbonate to remove traces of acid. The solution of the product is then filtered to remove insoluble impurities and the product recovered from the filtrate by evaporation of solvent leaving substantially pure product as a solid residue.

Compound II hereinabove is then treated with an amine compound to replace the chloro-substituent as illustrated hereinbelow:

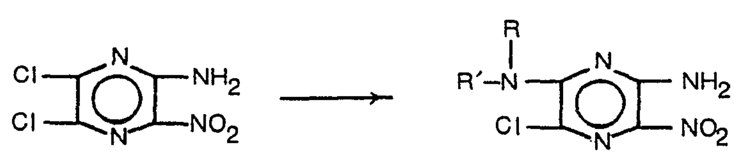

II III

The reaction of the dichloro-nitropyrazine II with the amine reagent is preferably carried out in a solvent for the reaction. The solvent is a lower alkanol such as methanol, ethanol or isopropanol; an ether such as tetrahydrofuran; dimethyl formamide; or acetonitrile. The amine reactant is preferably present in approximately a 5—10% molar excess over the nitropyrazine. The reagents are first mixed and then preferably heated to a temperature of 20—89°C for a period of from 0.5 to 24 hours. In addition to the reagents and the solvent it is generally preferred to carry out the reaction in the presence of at least an equimolar amount of an organic base. Tertiary amines such as triethylamine and pyridine are preferred. The progress of the reaction is followed by the use of thin layer chromatography.

As indicated hereinabove acid addition salts of compounds of Formula I may be prepared by mixing a selected compound of Formula I with an equimolar amount of a strong mineral acid in a solvent for the reactants at a temperature of 0 to 50°C. Solvents preferred are lower alkanols such a methanol, ethanol and isopropanol. The salt is precipitated from the alcoholic solution, filtered, washed with a minimal amount of cold alcohol and air dried.

The starting 5,6-dichloro-3-nitropyranzamine IV, which has been prepared by heating 3-amino-5,6-dichloropyrazin-carboxylic acid with a mixture of fuming sulfuric and fuming nitric acids as described above, is diazotized and exchanged with halogen ion to produce the important novel intermediate 5,6-dichlor-2-halo (Cl, Br,I)-3-nitropyrazine VA, which in turn is treated with one or two moles of a suitably

substituted alkylamine AH to produce a 2-(alkylamino)-3-chloro-6-halo-5-nitropyrazine VIA or 3-chloro-2,6-di(alkylamino)-5-nitropyrazine IA as illustrated in the reaction scheme below.

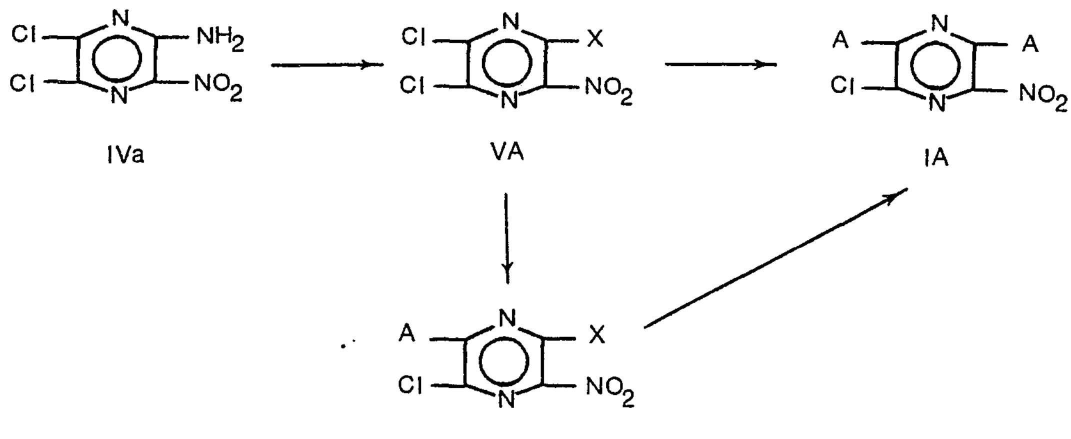

IVa VA IA

VIA

where A is as defined above and X is chloro, bromo or iodo. As shown in the flow sheet, intermediate compound VIA is isolated and reacted, if desired with a different species of alkylamine to produce a 2,6-disubstituted-3-chloro-5-nitropyrazine in which the 2 and 6 substituents differ from each other.

In carrying out the first step of the process, starting material IVA is diazotized and exchanged with halogen ion, e.g. chlorine, bromine or iodine, to produce the desired 2-halo-5,6-dichloro-3-nitropyrazine VA. In the preferred instance, compound IVA (5,6-dichloro-3-nitropyrazinamine) is dissolved in bromoform and to the solution is added at least one mole of an alkyl nitrite compound such as isoamyl nitrite, butyl nitrite or ethyl nitrite. The nitrite reagent is preferably added in approximately a 50—200% molar excess and the reaction mixture is heated at a temperature of 50—120°C for a period of 5—40 hours until the reaction is essentially complete as determined by thin-layer chromatography. Following the completion of the reaction the desired product is isolated from the cooled reaction mixture by evaporation of the excess of bromoform solvent *in vacuo*, leaving the crude product as a residual oil which is further purified by chromatography over silica gel.

The intermediate compound VA is condensed with one or two moles of an alkylamine optionally substituted with one or more hydroxy or alkoxy groups to produce the desired product IA in one or more reaction steps with intermediate production, if desired, of the non-substituted pyrazine VIA. This reaction, which results in the replacement of the 2 and 6 halogen substituents by alkylamino substituents, is conducted by mixing together 2-halo-5,6,-dichloro-3-nitropyrazine with at least a 100% molar excess of the selected amine compound and maintaining the reaction temperature at 0—50°C for a period of from 0.5 to 16 hours. Preferably the reaction is conducted in the presence of a solvent for the reactants, e.g. a lower aliphatic alcohol such as methanol, ethanol or isopropanol; certain ethers such as tetrahydrofuran; dimethyl formamide, or acetonitrile. Progress of the reaction is conveniently followed by monitoring the disappearance of starting material and appearance of product using thin-layer chromatography. Following completion of the reaction the solvent is removed by evaporation *in vacuo* leaving product as a residual oil which is further purified by trituration with solvents such as ethanol/chloroform mixture followed by chromatography on silica gel.

The product of formula IA produced may be varied by varying the amine reagent. Thus, for example, reaction of 2-bromo-5,6-dichloronitropyrazine with one or two moles of selected amine reagent produces the corresponding nitropyrazine in which the 6-chloro and/or the 2-bromo substituent are/is replaced by an amino substituent.

One preferred sub-group of active compounds is represented by the formula:

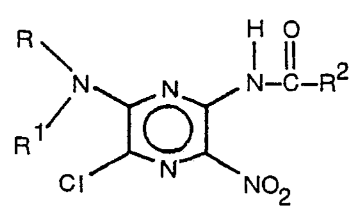

IIB

where each of R and $R^1$ is hydrogen, $C_{1-6}$ alkyl, $C_{1-6}$ substituted alkyl having one or more amino, $C_{1-6}$ alkylamino, di($C_{1-6}$ alkyl)amino, $C_{1-6}$ alkoxy or hydroxy substituents, $C_{2-6}$ alkenyl, or $C_{2-6}$ substituted alkenyl having one or more amino, $C_{1-6}$ alkylamino, di($C_{1-6}$ alkyl)amino, $C_{1-6}$ alkoxy or hydroxy substituents, or R and $R^1$ together form a morpholine, piperazine or N-substituted piperazine ring in which the N-substituent is hydrogen, $C_{1-6}$ hydroxyalkyl or $C_{1-6}$ alkoxyalkyl; and $R^2$ is $C_{1-6}$ alkyl.

The acylaminopyrazine described hereinabove are prepared by acylation and alkylation of the corresponding 5,6-dichloro-3-nitropyrazinamine, preparation of which is described hereinabove.

The 5,6-dichloro-3-nitropyrazinamine is acylated by treatment with an acid halide or an acid anhydride of a lower aliphatic carboxylic acid. Preferably, the 5,6-dichloro-3-nitropyrazinamine is treated at room temperature with a large excess of an acid halide, for example, acetyl chloride, acetyl bromide, propionyl chloride or butyryl chloride in the presence of a base such as sodium bicarbonate. The reaction is preferably conducted by mixing the reactants without added solvent at room temperature and heating the reactants at reflux temperature of the mixture for a period of 5 hours to 4 days to ensure maximum yield of product. Following the reaction period, the entire mixture is evaporated *in vacuo* to remove excess of acylating agent or alternatively quenched in water and extracted with a solvent for the product. After removal of volatile material by evaporation *in vacuo* the product remains as a residue and is conveniently further purified by chromatography over silica gel.

The 2-acylamino-5,6-dichloro-3-nitropyrazine thus produced is then treated with an amine compound to replace the chloro-substituent as illustrated below:

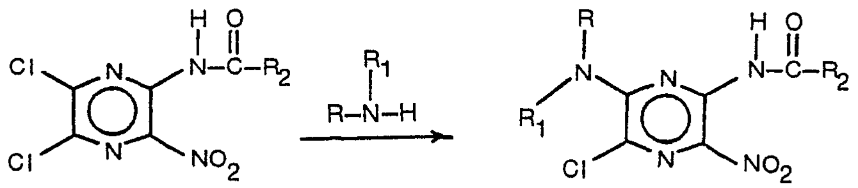

IIIB

IIB

where R, $R_1$ and $R_2$ are as defined in Compound IB hereinabove.

The reaction of the dichloroacylamino nitropyrazine IIIB with the amine compound is preferably carried out in a solvent for the reactants, e.g. a lower alkanol such as methanol, ethanol or isopropanol; an ether such as tetrahydrofuran; a N,N-dialkylamide such as dimethyl formamide; or acetonitrile. The amine or thiol reagent is preferably present in approximately a 5—10% molar excess over the nitropyrazine. The reagents are first mixed and then preferably heated to a temperature of 20—80°C for a period of from 0.5 to 24 hours. In addition to the reagents and the solvent it is generally preferred to carry out the reaction in the presence of at least an equimolar amount of an organic base. Tertiary amines such as triethylamine and pyridine are preferred. The progress of the reaction is followed by the use of thin-layer chromatography.

Following the reaction the product is recovered after first evaporating volatile solvents and reactants *in vacuo* leaving the product as a residue. This residue is then dissolved in a water-immiscible solvent such as ethyl acetate and the resulting extract of product is washed with water and evaporated *in vacuo* to produce substantially pure product.

The method of treatment of human patients or domestic animals undergoing radiation treatment of malignant disease uses the compound in pharmaceutical compositions that are administered orally or parentally, preferably intravenously. The dose depends on the radiation protocol for each individual patient. In protocols where the radiation dose is divided into a large number of fractions, the drug can be administered at intervals in the schedule and not necessarily with each radiation treatment. In general, the drug is administered from 10 minutes to 5 hours prior to the radiation treatment in a dosage amount of between 0.25 and about 4.0 grams per square meter of body surface.

The preferred dosage form for intravenous administration is a sterile isotonic solution of the drug. Oral dosage forms such as tablets, capsules, e.g. those containing from 100 to 500 mg of drug/capsule, or elixirs may also be used.

The following examples are intended to illustrate but not limit the invention.

Example 1—1

2-[(6-Amino-3-chloro-5-nitropyrazin-2-yl)amino]ethanol

Step A: Preparation of 5,6-dichloro-3-nitro pyrazinamine (II)

To 450 ml concentrate sulfuric acid cooled to 10°C is added 50.0 g (0.2 m) 3-amino-5,6-dichloropyrazine carboxylic acid. To this solution, cooled to 0—5°, is added a cold solution of 15 ml fuming sulfuric acid in 15 ml fuming nitric acid dropwise over 15 minutes. The reaction mixture is stirred at 0—5° for 2 hours and then at ambient temperature for 2 hours. The reaction mixture is then poured onto ice and the yellow solid is collected. This is taken up in ethyl acetate and the solution is washed twice with saturated aqueous sodium carbonate and filtered through a pad of silica gel. The resulting solution is evaporated *in vacuo* to afford 35 g of 5,6-dichloro-3-nitropyrazinamine, m.p. 169—170°C.

Step B: Preparation of 2[(6-Amino-3-chloro-5-nitropyrazin-2-yl)amino] ethanol

To 1.0 g of 5,6-dichloro-3-nitropyrazinamine in 20 ml isopropanol is added 0.5 ml triethylamine and then 0.3 ml 2-aminoethanol. The reaction mixture is stirred for 3 hours and then concentrated *in vacuo* to solids. The solids are triturated with hot chloroform and recystallized from acetonitrile to afford 0.6 g of 2[(6-amino-3-chloro-5-nitropyrazin-2-yl)amino] ethanol, m.p. 196—197°C.

By the procedure of Example 1—1, but using in Step B the appropriate amine, there are produced:

| Example | Reagent | Product | m.p. |
|---|---|---|---|
| 2 | diethanolamine | 3-chloro-2,6-di(2-hydroxyethylamino)-5-nitropyrazine | 194—196°C |
| 3 | 2-amino-2-methyl-1,3-propanediol | 2-[(6-amino-3-chloro-5-nitropyrazin-2-yl-amino]-2-methyl-1,3-propanediol | 214—215°C |
| 4 | 3-amino-1,2-propanediol | 3-[(6-amino-3-chloro-5-nitropyrazin-2-yl)-propanediol | 202—204°C |
| 5 | morpholine | N-(6-amino-3-chloro-5-nitro-pyrazin-2-yl)-morpholine | 211—212°C |
| 6 | N-methyl-ethanolamine | 6-amino-3-chloro-2-(N-methyl-N-(2-hydroxyethyl)-amino]-5-nitropyrazine | 166—167°C |
| 7 | Ethyl 3-amino crotonate | Ethyl 3-[(6-amino-3-chloro-5-nitropyrazin-2-yl)amino]crotonate | 162—163°C |
| 8 | 4-Methyl piperazine | 5-Chloro-6-(4-methyl-1-piperazinyl)-3-nitropyrazine amine | 186—188°C |
| 9 | 2-(dimethylamino) ethyl amine | 5-Chloro-6-[2-(dimethylamino)-ethyl amino)-3-nitropyrazine amine | |

Example 1—2

2,6-Di-(2-hydroxyethylamino)-3-chloro-5-nitropyrazine

Step A: 2-Bromo-5,6-dichloronitroyrazine

To 4.8 g isoamylnitrite in 150 ml bromoform heated to 95—100°C is added portion-wise 5.0 g 5,6-dichloro-3-nitropyrazinamine. The reaction mixture is heated at reflux for 18 hours. After cooling, the bromoform is removed *in vacuo* to give an oil which is chromatographed on silica gel. The initial component to elute is 5,6-dichloro-2-bromo-nitropyrazine, which is obtained (2.6 g) as a viscous oil.

Step B: Preparation of 2,6-di-(2-hydroxyethylamino)-3-chloro-5-nitropyrazine

To a solution of 1.0 g 2-bromo-5,6-dichloronitropyrazine in 10 ml isopropanol at 0—10°C is added 0.72 g triethylamine and then 0.44 g 2-aminoethanol. The reaction mixture is stirred at 0—10°C for 0.5 hour and then at room temperature for 0.5 hour. The solvent is then removed *in vacuo* and the residue triturated with ethanol/chloroform solution to afford a yellow solid which is collected by filtration. This solid is chromatographed on silica gel and the initial compound to be eluted is 2,6-di-(2-hydroxyethylamino)-3-chloro-5-nitro-pyrazine, 0.5 g, m.p. 148—150°C.

Step $B_1$: 2-[(6-Bromo-3-chloro-5-nitropyrazin-2-yl)-amino] ethanol

To 1.8 g of 2-bromo-5,6-dichloronitropyrazine in 10 ml isopropanol at 0—10°C is added 0.6 g triethylamine and then 0.31 g ethanolamine. The reaction mixture is stirred at 0—10°C for 0.5 hour and then

at room temperature for 15 minutes. The solvent is then removed *in vacuo* and the resulting oil chromatographed on silica gel with elution by 2% methanol/chloroform. The desired product is collected as a clear oil, identity confirmed by mass spectral analysis. This oil is treated with 0.31 g of ethanolamine and 0.6 g of triethylamine to form 2,2'-[(3-chloro-5-nitropyrazin-2,6-yl)diamino])-diethanol.

By the procedure of Example 1—2B but using in Step B the appropriate reagent, there is produced the product shown:

Example 2—2

| REAGENT | PRODUCT |
|---|---|
| 3-amino-1,2-propanediol | 3-chloro-2,6-di)2,3-dihydroxypropylamino)-5-nitropyrazine |

Example 3—2

| REAGENT | PRODUCT |
|---|---|
| Diethylamine | 3-chloro-2,6-diethylamino 5-nitropyrazine |

Example 1—3

N-[5-chloro-6-(2,3-dihydroxypropyl)amino-3-nitropyrazinyl]acetamide

Step A: Preparation of N-(5,6-dichloro-3-nitropyrazin-2-yl)acetamide

To 1.0 g of 5,6-dichloro-3-nitropyrazinamine in 15 ml acetyl chloride unde nitrogen atmosphere at room temperature is added 0.84 g anhydrous sodium bicarbonate. The reaction mixture is then stirred and heated at reflux for 4 days. Excess of acetyl chloride is then removed *in vacuo* and the residue is chromatographed on silica gel. The desired product is eluted from the column with chloroform.

Step B: N-[5-chloro-6-(2,3-dihydroxypropyl)amino-3-nitropyrazinyl]acetamide

To 0.20 g of acetamide in 35 ml isopropanel at room temperature is added 0.8 g triethylamine followed by 0.73 g 3-amino-1,2-propanediol. The reaction mixture is stirred at room temperature for 1 hour. Then, the solvent is removed *in vacuo* and the residue taken up in ethyl acetate and washed twice with 10 ml water. The dried ethyl acetate extract is stripped *in vacuo* to give yellow solid, which is recrystallized from acetonitrile to give the title compound, m.p. 130—131°C.

By the procedure of Example 1—2, Step B but using an equivalent amount of the indicated appropriate reagent, there is produced:

| Example | Reagents | Product |
|---|---|---|
| 2—3 | 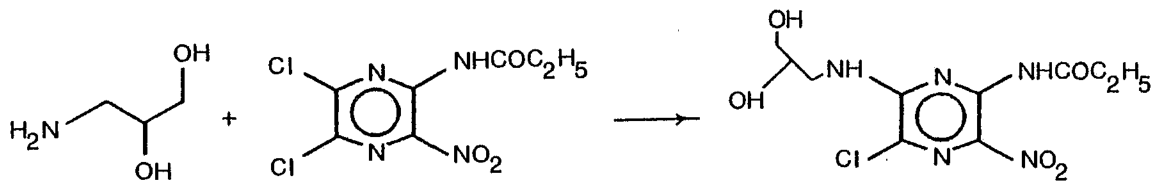 | N-[5-Chloro-6-(2,3-dihydroxypropyl)-amino-3-nitropyrazinyl]propionamide |
| 3—3 | 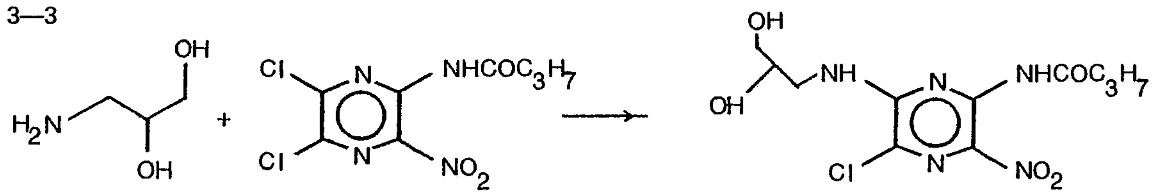 | N-[5-Chloro-6-(2,3-di-hydroxypropyl) amino-3-nitropyrazinyl]butyramide |
| 4—3 | 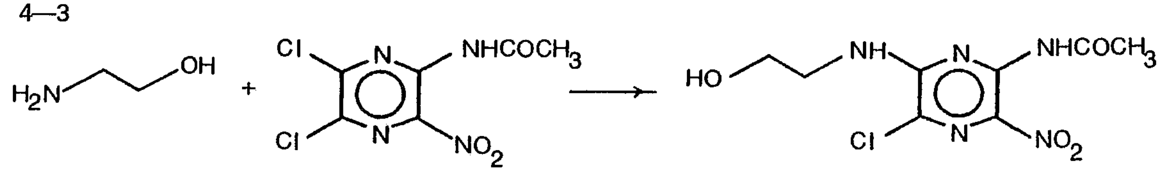 | N-[5-Chloro-6-(2-hydroxyethyl)amino-3-nitropyrazinyl]acetamide |

| Example | Reagents | Product |
|---|---|---|
| 5—3 | 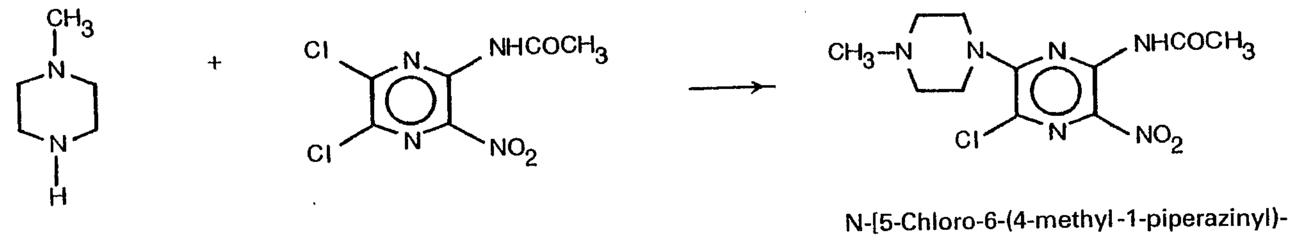 | N-[5-Chloro-6-(4-methyl-1-piperazinyl)-3-nitropyrazinyl]acetamide |
| 6—3 | 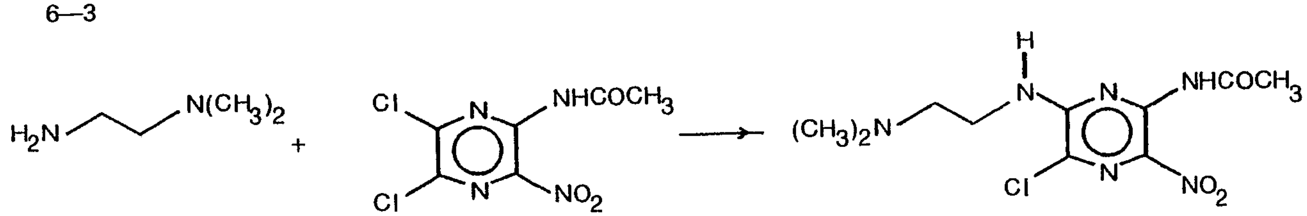 | N-[5-Chloro-6-(di-methylaminoethyl)-3-nitropyrazinyl]acetamide |

Example 7—3

Sterile Isotonic Solutions for Injection

Suitable formulations for injection are prepared by dissolving each of the compounds of the preceding Examples in isotonic solution in a concentration of about 1 mg/ml and sterilizing the resulting solution. They are suitable for intravenous injection.

Example 8—3

Capsules

Suitable formulations for oral administration are prepared by filling appropriately sized capsules individually with 100 and 500 mg portion of each of the compounds produced in accordance with the preceding Examples.

**Claims**

1. A pharmaceutical composition useful in enhancing the therapeutic effect of radiation treatment comprising a radiation-enhancing compound of the formula:

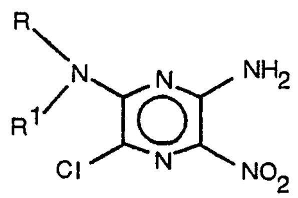

where each of R and $R^1$ is hydrogen, $C_{1-6}$ alkyl, $C_{2-6}$ alkenyl, $C_{1-6}$ substituted alkyl or $C_{2-6}$ substituted alkenyl having one or more halo, amino, $C_{1-6}$ alkylamino, di($C_{1-6}$ alkyl)amino, $C_{1-6}$ alkoxy, hydroxy, ethoxycarbonyl, phenyl, pyridyl, pyrimidinyl or imidazolyl substituents, or R and $R^1$ together form a morpholine, piperazine or N-substituted piperazine ring in which the N-substituent is hydrogen, $C_{1-6}$ alkyl, $C_{1-6}$ hydroxyalkyl or $C_{1-6}$ alkoxyalkyl; or a compound that is a pharmaceutically acceptable acid-addition salt thereof, and a pharmaceutical carrier.

2. For use in enhancing the therapeutic effect of radiation treatment, a compound as defined in Claim 1.

3. A composition useful in enhancing the therapeutic effect of radiation treatment comprising a compound having the formula:

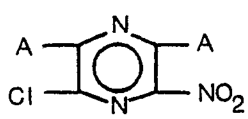

where A is an amino substituent having at least one of the hydrogens of the amino groups replaced by a hydroxy-substituted or ($C_{1-6}$ alkoxy)-substituted $C_{1-6}$ alkyl residue, or a compound that is pharmaceutically acceptable acid-addition salt thereof, and a pharmaceutical carrier.

4. For use in enhancing the therapeutic effect of radiation treatment, a compound as defined in Claim 3.

5. A pharmaceutical composition useful in enhancing the therapeutic effect of radiation treatment comprising an effective amount of radiation enhancing compound of the formula:

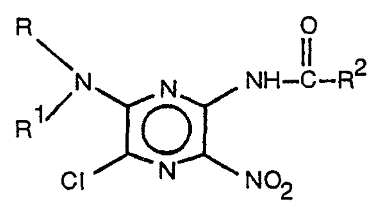

where each of R and $R^1$ is hydrogen, $C_{1-6}$ alkyl, $C_{1-6}$ substituted alkyl having one or more amino, $C_{1-6}$ alkylamino, di($C_{1-6}$ alkyl)amino, $C_{1-6}$ alkoxy or hydroxy substituents, $C_{2-6}$ alkenyl, or $C_{2-6}$ substituted alkenyl having one or more amino, $C_{1-6}$ alkylamino, di($C_{1-6}$ alkyl)amino, $C_{1-6}$ alkoxy or hydroxy substituents, or R and $R^1$ together form a morpholine, piperazine or N-substituted piperazine ring in which the N-substituent is hydrogen, $C_{1-6}$ alkyl, $C_{1-6}$ hydroxyalkyl or $C_{1-6}$ alkoxyalkyl; and $R^2$ is $C_{1-6}$ alkyl; or a compound that is pharmaceutically acceptable acid-addition salt thereof, and a pharmaceutical carrier.

6. For use in enhancing the therapeutic effect of radiation treatment, an effective sensitizing amount of a compound as defined in Claim 5.

**Patentansprüche**

1. Zur Erhöhung des therapeutischen Effekts einer Strahlenbehandlung verwendbare pharmazeutische Zusammensetzung, enthaltend eine Strahlen-verstärkende Verbindung der Formel

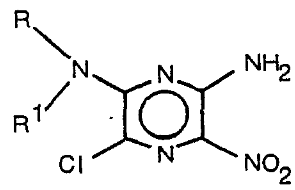

worin jedes von R und $R^1$ Wasserstoff, $C_{1-6}$-Alkyl, $C_{2-6}$-Alkenyl, $C_{1-6}$ substituiertes-Alkyl oder $C_{2-6}$-substituiertes-Alkenyl mit einem oder mehreren Halogen-, Amino-, $C_{1-6}$-Alkylamino-, Di($C_{1-6}$-alkyl)-amino-, $C_{1-6}$-Alkoxy, Hydroxy-, Ethoxycarbonyl-, Phenyl-, Pyridyl-, Pyrimidinyl- oder Imidazolyl- Substituenten ist oder R und $R^1$ zusammen einen Morpholin-, Piperazin- oder N-substituierten-Piperazinring bilden, worin der N-Substituent Wasserstoff, $C_{1-6}$-Alkyl, $C_{1-6}$-Hydroxyalkyl oder $C_{1-6}$-Alkoxyalkyl ist; oder eine Verbindung, welche ein pharmazeutisch annehmbares Säureadditionssalz davon ist, und einen pharmazeutischen Träger.

2. Zur Verwendung der Erhöhung des therapeutischen Effekts einer Strahlenbehandlung, eine Verbindung nach Anspruch 1.

3. Zur Erhöhung des therapeutischen Effekts einer Strahlenbehandlung verwendbare Zusammensetzung, enthaltend eine Verbindung mit der Formel

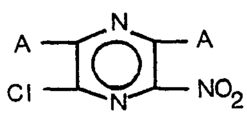

worin A ein Aminosubstituent ist, in welchem wenigstens einer der Wasserstoffe der Aminogruppen durch einen Hydroxy-substituierten oder ($C_{1-6}$-Alkoxy)-substituierten $C_{1-6}$-Alkylrest ersetzt ist, oder eine Verbindung, welche ein pharmazeutisch annehmbares Säureadditionssalz davon ist, und einen pharmazeutischen Träger.

4. Zur Verwendung der Erhöhung des therapeutischen Effekts einer Strahlenbehandlung, eine Verbindung nach Anspruch 3.

5. Zur Erhöhung des therapeutischen Effekts einer Strahlenbehandlung verwendbare pharmazeutische Zusammensetzung, enthaltend eine wirksame Menge einer Strahlen-verstärkenden Verbindung der Formel

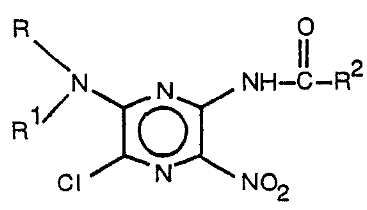

worin jedes von R und $R^1$ Wasserstoff, $C_{1-6}$-Alkyl, $C_{1-6}$-substituiertes-Alkyl mit einem oder mehreren Amino-, $C_{1-6}$-Alkylamino-, Di($C_{1-6}$-alkyl)amino-, $C_{1-6}$-Alkoxy oder Hydroxysubstituenten, $C_{2-6}$-Alkenyl oder $C_{2-6}$-substituiertes-Alkenyl mit einem oder mehreren Amino-, $C_{1-6}$-Alkylamino-, Di($C_{1-6}$-alkyl)amino-, $C_{1-6}$-Alkoxy, oder Hydroxysubstituenten ist oder R und $R^1$ zusammen einen Morpholin-, Piperazin- oder N-substituierten-Piperazinring bilden, in welchem der N-Substituent Wasserstoff, $C_{1-6}$-Alkyl, $C_{1-6}$-Hydroxyalkyl oder $C_{1-6}$-Alkoxyalkyl ist; und $R^2$ $C_{1-6}$-Alkyl ist; oder eine Verbindung, welche ein pharmazeutisch annehmbares Säureadditionssalz davon ist, und einen pharmazeutischen Träger.

6. Zur Verwendung zur Verstärkung des therapeutischen Effekts einer Strahlenbehandlung, eine wirksam sensibilisierende Menge einer Verbindung nach Anspruch 5.

**Revendications**

1. Une composition pharmaceutique utile pour accroître l'effet thérapeutique d'un traitement par des radiations comprenant un composé accroissant l'effet des radiations de formule:

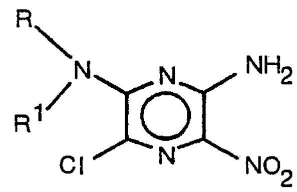

dans laquelle chacun de R et $R^1$ est un hydrogène, un alkyle en $C_{1-6}$, un alcényle en $C_{2-6}$, un alkyle en $C_{1-6}$ substitué ou un alcényle en $C_{2-6}$ substitué ayant un ou plusieurs substituants halogéno, amino, alkylamino en $C_{1-6}$, di(alkyl en $C_{1-6}$) amino, alcoxy en $C_{1-6}$, hydroxy, éthoxycarbonyle, phényle, pyridyle, pyrimidinyle ou imidazole, ou R et $R^1$ forment ensemble un cycle morpholine, pipérazine ou pipérazine N-substituée dont le substituant à l'azote est un hydrogène, un alkyle en $C_{1-6}$, un hydroxyalkyle en $C_{1-6}$ ou un alcoxyalkyle en $C_{1-6}$; ou un composé qui est un sel d'addition d'acide convenant en pharmacie de celui-ci, et un support pharmaceutique.

2. Pour l'emploi pour accroître l'effet thérapeutique d'un traitement par des radiations, un composé comme défini dans la revendication 1.

3. Une composition utile pour accroître l'effet thérapeutique d'un traitement par des radiations comprenant un composé ayant pour formule:

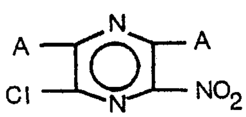

dans laquelle A est un substituant amino ayant au moins un des hydrogènes des groupes amino remplacé par un reste alkyle en $C_{1-6}$ hydroxy-substitué ou (alcoxy en $C_{1-6}$)-substitué ou un composé qui est un sel d'addition d'acide convenant en pharmacie de celui-ci et un support pharmaceutique.

4. Pour l'emploi pour accroître l'effet thérapeutique d'un traitement par des radiations, un composé comme défini dans la revendication 3.

5. Une composition pharmaceutique utile pour accroître l'effet thérapeutique d'un traitement par des radiations comprenant une quantité efficace d'un composé accroissant l'effet des radiations de formule:

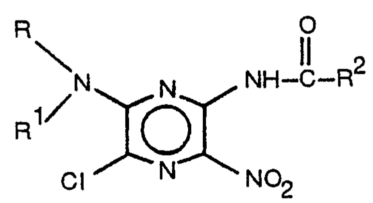

dans laquelle chacun de R et $R^1$ est un hydrogène, un alkyle en $C_{1-6}$, un alkyle en $C_{1-6}$ substitué ayant un ou plusieurs substituants amino, alkylamino en $C_{1-6}$, di(alkyl en $C_{1-6}$)amino, alcoxy en $C_{1-6}$ ou hydroxy, ou R et $R^1$ ensemble forment un cycle morpholine, pipérazine ou pipérazine N-substituée dont le substituant à l'azote est un hydrogène, un alkyle en $C_{1-6}$, un hydroxyalkyle en $C_{1-6}$ ou un alcoxyalkyle en $C_{1-6}$ et $R^2$ est un alkyle en $C_{1-6}$; ou un composé qui est un sel d'addition d'acide convenant en pharmacie de celui-ci, et un support pharmaceutique.

6. Pour l'emploi pour accroître l'effet thérapeutique d'un traitement par des radiations, un composé comme défini dans la revendication 5.